# EUROPÄISCHE PATENTANMELDUNG

(11) **EP 4 721 807 A1**
(43) Veröffentlichungstag der Anmeldung: **08.04.2026**
(21) Anmeldenummer: 24203798.4
(22) Anmeldetag: 01.10.2024
(51) Int. Cl.: A61M 39/20, A61M 25/00, A61M 39/08

(54) **VORRICHTUNG UND VERFAHREN ZUM SCHNEIDEN UND VERSCHLIESSEN EINES MEDIZINISCHEN SCHLAUCHS**

(71) Anmelder: Heraeus Medical GmbH, 61273 Wehrheim (DE)
(72) Erfinder: Warmbier, Moritz, 61273 Wehrheim (DE); Vogt, Sebastian, 61273 Wehrheim (DE); Stangl, Roland, 85368 Moosburg (DE); Youzbachi, Said, 80331 München (DE); Rokitta, Hermann Josef, 45481 Mülheim an der Ruhr (DE)
(74) Vertreter: Heraeus IP

(57) **Zusammenfassung**

Die Erfindung betrifft eine Vorrichtung zum Verschließen, bevorzugt zum Schneiden und Verschließen eines medizinischen Schlauchs. Die Erfindung betrifft ferner eine zugehörige Verwendung sowie ein Verfahren. Eine Vorrichtung (10) zum Verschließen eines medizinischen Schlauchs (1) umfasst eine Halteeinrichtung (12) zum Halten eines medizinischen Schlauchs (1), eine Aufbewahrungseinrichtung (20) zum Aufbewahren eines Schlauchverschlusses (7) sowie eine Verschlusseinrichtung (22) zum Verschließen des in der Halteeinrichtung (12) gehaltenen Schlauchs (1) mit einem in der Aufbewahrungseinrichtung (20) befindlichen Schlauchverschluss (7).

## Beschreibung

Die Erfindung betrifft eine Vorrichtung zum Verschließen, bevorzugt zum Schneiden und Verschließen eines medizinischen Schlauchs. Die Erfindung betrifft ferner eine zugehörige Verwendung sowie ein Verfahren.

Bei bestimmten Diagnosen wie Osteomyelitis / Osteitis ist es nach erfolgter chirurgischer Sanierung (Debridement) zielführend, neben einer systemischen Antibiose auch lokal Wirkstoffe wie Antibiotika anzuwenden. So kann eine Reinfektion des Gewebes verhindert werden. Systeme zur lokalen Wirkstofffreisetzung umfassen Kollagenschwämme, Polymethylmethacrylat-Kugelketten, Polymethylmethacrylat-Spacer und Elemente aus Calciumsulfat, die Wirkstoffe wie Antibiotika, z. B. Gentamicin, enthalten. Typischerweise sind die Wirkstoffe wasserlöslich und werden durch Einwirkung wässriger Körperflüssigkeiten wie Blut und Wundsekret herausgelöst. Die Freisetzung erfolgt durch Diffusion. Deshalb ist die anfängliche Konzentration sehr hoch und fällt mit der Zeit stark ab. Wünschenswert wäre jedoch eine kontinuierliche, etwa gleichmäßige Wirkstoff-Freisetzung über einen Zeitraum von Tagen bis Wochen.

Um dies zu ermöglichen, schlägt die Druckschrift EP 3 795 196 B1 ein System zur lokalen Wirkstofffreisetzung vor, welches einen Schlauch mit einer Vielzahl schlitzförmiger Ventile umfasst. Die Ventile öffnen sich beim Einpressen von Flüssigkeit in den Schlauch ab einem bestimmten Grenzdruck und ermöglichen eine gleichmäßige Freisetzung der Flüssigkeit über die jedes Ventil bzw. über die gesamte Schlauchlänge. Beim Nachlassen der Druckbeaufschlagung verschließen sich die Ventile wieder. So ist eine wiederholte Freisetzung von Flüssigkeit durch den Schlauch mit den sich druckabhängig selektiv öffnenden und schließenden Ventilen möglich. Die Flüssigkeit enthält beispielsweise eine Lösung, Suspension und/oder Emulsion mindestens eines Wirkstoffs.

Das im Patienten befindliche erste Ende des Schlauchs ist dabei mit einem Stopfen verschlossen. So wird ein unkontrolliertes Auslaufen der Flüssigkeit verhindert. Der Stopfen muss dem Überdruck im Schlauch sicher standhalten. In das gegenüberliegende, außerhalb des Patienten befindliche zweite Ende des Schlauchs wird die Flüssigkeit eingeleitet. Die Ventile sind üblicherweise von dem ersten Ende bis zu einer mittigen Position des Schlauchs angeordnet.

Allerdings sind die zu behandelnden Knochen- und Weichgewebeareale bei jedem Patienten unterschiedlich groß. Deshalb besteht die Notwendigkeit, den Schlauch hinsichtlich seiner Länge anzupassen. Die Anpassung ist grundsätzlich durch Schneiden des Schlauchs möglich. Üblicherweise wird dabei das erste Ende des Schlauchs abgeschnitten und so ein neues erstes Ende hergestellt. Es sind dann immer noch bis zum (neuen) ersten Ende des Schlauchs Ventile vorhanden. Der verkürzte Schlauch muss anschließend wieder mit einem Stopfen verschlossen werden. Bei den üblichen Schlauchdurchmessern von ≤ 4 mm hat der Stopfen typischerweise einen Außendurchmesser von < 3 mm. Es ist wünschenswert, das Kürzen und Verschließen intraoperativ durchzuführen, um anhand der individuellen Anatomie des Patienten die optimale Länge des Schlauchs einzustellen.

Eine sichere und druckfeste Platzierung des sehr kleinen Stopfens ist manuell sehr schwer, insbesondere unter OP-Bedingungen, z. B. mit Gummihandschuhen. Der Stopfen kann herunterfallen. Zudem ist es möglich, dass der Stopfen nicht vollständig eingepresst wird und es zu Undichtigkeiten kommt.

Es ist die Aufgabe der Erfindung, ein einfaches und sicheres Setzen des Stopfens zu ermöglichen, insbesondere unter OP-Bedingungen.

Die Aufgabe wird gelöst durch die Vorrichtung gemäß Anspruch 1 sowie die Verwendung und das Verfahren gemäß den nebengeordneten Ansprüchen. Vorteilhafte Ausgestaltungen sind in den Unteransprüchen angegeben.

Zur Lösung der Aufgabe dient eine Vorrichtung zum Verschließen eines medizinischen Schlauchs. Die Vorrichtung kann eine Halteeinrichtung zum Halten eines medizinischen Schlauchs umfassen. Die Vorrichtung kann eine Aufbewahrungseinrichtung zum Aufbewahren eines Schlauchverschlusses umfassen. Die Vorrichtung kann eine Verschlusseinrichtung zum Verschließen des in der Halteeinrichtung gehaltenen Schlauchs mit einem in der Aufbewahrungseinrichtung befindlichen Schlauchverschluss umfassen.

Die Vorrichtung erlaubt ein Verschließen eines schlauchförmigen Systems zur lokalen Wirkstofffreisetzung, insbesondere ein intraoperatives Verschließen.

Die Halteeinrichtung hält den Schlauch, während er mit dem Schlauchverschluss verschlossen wird. Insbesondere erfolgt ein Halten gegen ein axiales Verschieben. So muss kein manuelles Halten erfolgen und das Halten ist sicherer und einfacher möglich. Das Halten erfolgt insbesondere durch kraftschlüssigen und/oder formschlüssigen Kontakt eines oder mehrerer Teile der Halteeinrichtung mit der Außenwand des Schlauchs. Mit anderen Worten fixiert die Halteeinrichtung den Schlauch.

Durch die Aufbewahrungseinrichtung kann der Schlauchverschluss sicher aufbewahrt und/oder gehalten werden. Ein Heraus- oder Herunterfallen wird verhindert. In einem einfachen Beispiel hält die Aufbewahrungseinrichtung den Schlauch außen oder innen an der Vorrichtung, so dass dieser nicht verloren geht. Insbesondere ist die Aufbewahrungseinrichtung zum kraftschlüssigen und/oder formschlüssigen Halten des Schlauchverschlusses eingerichtet. Beispielsweise umfasst die Aufbewahrungseinrichtung einen Hohlraum zur Aufnahme des Schlauchverschlusses. In einer Ausführungsform kann der Schlauchverschluss in den Schlauch gepresst werden, ohne dass der Benutzer in Kontakt mit dem Schlauchverschluss gelangt, beispielsweise durch Bewegen aus der Aufbewahrungseinrichtung heraus direkt in das Ende des Schlauchs. So eine Kontamination des Schlauchverschlusses verhindert werden. Die Vorrichtung kann den Schlauchverschluss umfassen, insbesondere in der Aufbewahrungseinrichtung. Der Schlauchverschluss ist insbesondere aus Kunststoff und/oder Gummi hergestellt.

In einer Ausführungsform ist der Schlauchverschluss ein Stopfen, der in das Ende des Schlauchs eingepresst werden kann. Ein Einpressbereich des Stopfens, der sich nach dem Einpressen im Inneren des Schlauchs befindet, kann zumindest bereichsweise ein Übermaß aufweisen. So wird eine besonders feste Verbindung zwischen Schlauch und Stopfen möglich. Der Einpressbereich kann zumindest bereichsweise eine kreiszylinderförmige Grundform aufweisen. Beispielsweise kann der Einpressbereich einen zylinderförmigen Abschnitt mit einem vergrößerten Durchmesser aufweisen, der größer ist als der Innendurchmesser des Schlauchs. In Längsrichtung benachbart zu dem vergrößerten Bereich kann ein Bereich mit einem kleineren Durchmesser vorhanden sein, der etwa dem Innendurchmesser des Schlauchs entsprechen kann. Alternativ kann auch der gesamte Einpressbereich den vergrößerten Durchmesser aufweisen. Es kann ein konischer Abschnitt vorhanden sein, beispielsweise im vorderen Bereich, der zuerst in den Schlauch eingepresst wird. Dies kann das Einpressen erleichtern und/oder sicherer machen. Es kann einen oder mehrere konische Bereiche geben, die weiter hinten angeordnet sind. Der Stopfen kann eine oder mehrere Formschlusselemente aufweisen, beispielsweise nach hinten und/oder außen weisende Rastelemente oder Haken, die in die Innenwand des Schlauchs greifen und so einen formschlüssigen Schutz gegen ein Herausbewegen des Stopfens aus dem Schlauch bieten. Es können mehrere Formschlusselemente in Umfangsrichtung verteilt angeordnet sein. Insbesondere ragen die Formschlusselemente radial weniger über die benachbarte Außenfläche des Stopfens hinaus nach außen als die Wandstärke des Schlauchs, typischerweise weniger als die Hälfte der Wandstärke. So kann eine Beschädigung des Schlauchs verhindert werden.

Die Verschlusseinrichtung verschließt den Schlauch. So muss dies nicht manuell erfolgen und das Verschließen wird sicherer und einfacher. Die Verschlusseinrichtung ist bevorzugt dazu eingerichtet, einen Schlauch gemäß EP 3 795 196 B1 zu verschließen. Die Verschlusseinrichtung ist insbesondere dazu eingerichtet, den Schlauch zu verschließen, während er von der Halteeinrichtung gehalten wird.

In einer Ausgestaltung weist die Vorrichtung ferner eine Trennvorrichtung zum Durchtrennen des Schlauchs auf. Insbesondere ist die Verschlusseinrichtung dazu eingerichtet, ein mit der Trennvorrichtung hergestelltes Ende des Schlauchs zu verschließen.

Auf diese Weise kann auch das Kürzen des Schlauchs besonders einfach und sicher erfolgen. Es ist kein manuelles Kürzen, beispielsweise mit Schere oder Messer, nötig. Mögliche Fehler, wie schräge oder unsaubere Schnitte oder Kontamination, sowie Verletzungsgefahr aufgrund der verwendeten Werkzeuge werden verhindert. Der Einsatz des medizinischen Schlauchs wird wesentlich vereinfacht, weil nur eine einzige Vorrichtung zum Kürzen und Verschließen des Schlauchs benötigt wird.

Die Trennvorrichtung umfasst insbesondere eine Klinge zum Schneiden des Schlauchs. So kann ein einfaches und definiertes Durchtrennen erfolgen. Die Klinge ist insbesondere im Inneren der Trennvorrichtung angeordnet, um Verletzungen auszuschließen. Die Klinge kann schräg angeordnet sein, beispielsweise bezogen auf eine Bewegungsrichtung der Klinge, um einen leichten und gleichmäßigen Schnitt bei konstanter Kraft zu ermöglichen. Es kann aber auch jede beliebige andere Trennvorrichtung verwendet werden, beispielsweise ein heißer Draht, ein Laser, etc.

Insbesondere kann das hergestellte Ende verschlossen werden. Bevorzugt muss der Schlauch nur einmal in Bezug zur Vorrichtung positioniert werden und anschließend kann das Durchtrennen und Verschließen erfolgen. Typischerweise können das Durchtrennen und Verschließen innerhalb kurzer Zeit, beispielsweise innerhalb von wenigen Sekunden, erfolgen. Das Durchtrennen kann erfolgen, ohne dass der Schlauch in der Halteeinrichtung gehalten wird, oder wenn der Schlauch in der Halteeinrichtung gehalten wird.

In einer Ausführungsform ist die Vorrichtung so eingerichtet, dass der Schlauch während des Durchtrennens von der Halteeinrichtung gehalten wird. So muss der Schlauch bevorzugt nur einmal gehalten bzw. gegriffen werden, um das Durchtrennen und das Verschließen zu ermöglichen. Es kann so ein besonders schnelles Durchtrennen und Verschließen erfolgen.

In einer Ausgestaltung weist die Verschlusseinrichtung ein Presselement zum Pressen des Schlauchverschlusses auf oder in den Schlauch auf. Insbesondere ist das Presselement entlang einer Längsachse des Schlauchs beweglich.

Insbesondere ist die Verschlusseinrichtung dazu eingerichtet, den Schlauchverschluss in ein Ende des Schlauchs zu schieben bzw. zu pressen. Das Presselement kann dazu eingerichtet sein, den Schlauchverschluss aus der Aufbewahrungseinrichtung direkt entlang seiner Längsachse in Richtung des Schlauchs und/oder in den Schlauch hineinzubewegen. Das Presselement kann die Form einer Stange mit beliebigem Querschnitt aufweisen, wobei ein freies Ende der Stange als Pressfläche dient. Das Presselement kann sich entlang der Längsachse des Schlauchverschlusses erstrecken.

In einer Ausgestaltung weist die Vorrichtung eine erste Bewegungseinrichtung auf, wobei die Vorrichtung so eingerichtet ist, dass mit einer ersten Bewegung der ersten Bewegungseinrichtung das Halten des Schlauchs und/oder das Durchtrennen des Schlauchs ausgelöst wird.

Insbesondere umfasst die Vorrichtung einen Grundkörper. Die erste Bewegungseinrichtung kann in Bezug zum Grundkörper bewegt werden, und zwar typischerweise von einer Ausgangsposition in eine Zielposition. Die erste Bewegung ist bevorzugt eine lineare Bewegung. Die erste Bewegungseinrichtung kann also verschoben werden. Die erste Bewegung erfolgt insbesondere senkrecht zu einem Kanal der Vorrichtung und/oder zur Längsachse des Schlauchs. Die Längsachse des Schlauchs bezieht sich auf eine Stelle des Schlauchs im Bereich der Vorrichtung.

Insbesondere befindet sich der Schlauch innerhalb der Vorrichtung in einer geraden Ausrichtung.

Ein Teil der Halteeinrichtung kann mit der ersten Bewegungseinrichtung in Wirkverbindung stehen, beispielsweise Teil der ersten Bewegungseinrichtung sein. Die erste Bewegung der ersten Bewegungseinrichtung kann dazu führen, dass der Schlauch gehalten wird. Insbesondere erfolgt zuvor das Positionieren des Schlauchs in Bezug zur Vorrichtung, beispielsweise durch Einführen des Schlauchs in den Kanal. Wenn die Halteeinrichtung zwei Schenkel aufweist, zwischen denen der Schlauch kraftschlüssig und/oder formschlüssig gehalten werden kann, können die beiden Schenkel mit der ersten Bewegungseinrichtung verbunden sein und durch die erste Bewegung zum Schlauch bewegt werden, um den Schlauch nach der Bewegung zwischen den Schenkeln zu halten. Schenkel der Halteeinrichtung sind insbesondere senkrecht zur Längsachse des Kanals und/oder des Schlauchs ausgerichtet.

Die Trennvorrichtung kann mit der ersten Bewegungseinrichtung in Wirkverbindung stehen, beispielsweise Teil der ersten Bewegungseinrichtung sein. Die erste Bewegung der ersten Bewegungseinrichtung kann dazu führen, dass der Schlauch durchtrennt wird. Insbesondere erfolgt zuvor das Positionieren des Schlauchs in Bezug zur Vorrichtung, wie beschrieben. Wenn die Trennvorrichtung eine Klinge zum Durchtrennen des Schlauchs aufweist oder als Klinge ausgeführt ist, kann die Klinge mit der ersten Bewegungseinrichtung verbunden sein und durch die erste Bewegung zum Schlauch bewegt werden, um den Schlauch zu durchtrennen. Die Klinge ist insbesondere senkrecht zur Längsachse des Kanals und/oder des Schlauchs ausgerichtet.

Werden sowohl das Halten als auch das Durchtrennen durch die erste Bewegung ausgelöst, sind der Teil der Halteeinrichtung und der Teil der Trennvorrichtung bevorzugt so angeordnet, dass während der ersten Bewegung zuerst der Teil der Halteeinrichtung mit dem Schlauch in Kontakt kommt. So kann der Schlauch zuerst gehalten werden. Erst danach kommt der Teil der Trennvorrichtung mit dem Schlauch in Kontakt, um den gehaltenen Schlauch zu durchtrennen. Alternativ kann auch zuerst die Trennvorrichtung mit dem Schlauch in Kontakt kommen, um den Schlauch zu durchtrennen, bevor der Schlauch für ein Verschließen mittels der Halteeinrichtung gehalten wird. Es können auch beide Schritte, das Halten und das Schneiden, gleichzeitig erfolgen. Die Zielposition wird erst erreicht, wenn der Schlauch durchtrennt ist. Es werden zwei Schritte durch eine einzige Betätigung des Benutzers durchgeführt. So können das Halten und das Durchtrennen besonders einfach und schnell erfolgen.

Der Grundkörper umfasst typischerweise ein Gehäuse, das vom Benutzer manuell gegriffen werden kann. Der Grundkörper kann im Wesentlichen oder vollständig aus Kunststoff hergestellt sein. Der Grundkörper weist typischerweise eine Länge von wenigstens 8 cm, bevorzugt wenigstens 10 cm und/oder höchstens 30 cm, bevorzugt höchstens 25 cm auf. Der Grundkörper weist typischerweise eine Breite und/oder Höhe von wenigstens 3 cm, bevorzugt wenigstens 5 cm und/oder höchstens 20 cm, bevorzugt höchstens 15 cm auf. Der Grundkörper kann eine erste Ausnehmung aufweisen, in der die erste Bewegungseinrichtung zumindest teilweise aufgenommen werden kann. Der Grundkörper kann eine zweite Ausnehmung aufweisen, in der die zweite Bewegungseinrichtung zumindest teilweise aufgenommen werden kann.

Die Worte "erste", "zweite", etc. dienen lediglich zur begrifflichen Unterscheidung etwaig vorhandener mehrerer Gegenstände oder Schritte mit ansonsten gleicher Bezeichnung. Ist eine erste Bewegungseinrichtung vorhanden, muss nicht notwendigerweise eine zweite Bewegungseinrichtung vorhanden sein. Erfolgt eine zweite Bewegung, muss nicht notwendigerweise eine erste Bewegung erfolgt sein oder erfolgen.

In einer Ausgestaltung weist die Vorrichtung eine zweite Bewegungseinrichtung auf, wobei die Vorrichtung so eingerichtet ist, dass mit einer zweiten Bewegung der zweiten Bewegungseinrichtung das Verschließen des Schlauchs und/oder ein Freigeben des Schlauchs ausgelöst wird.

Die zweite Bewegungseinrichtung kann in Bezug zum Grundkörper bewegt werden, und zwar typischerweise von einer Ausgangsposition in eine Zielposition. Die zweite Bewegung ist bevorzugt eine lineare Bewegung. Die zweite Bewegungseinrichtung kann also verschoben werden. Die zweite Bewegung erfolgt insbesondere parallel zum Kanal der Vorrichtung und/oder zur Längsachse des Schlauchs.

Ein Teil der Verschlusseinrichtung, insbesondere das Presselement, kann mit der zweiten Bewegungseinrichtung in Wirkverbindung stehen, beispielsweise Teil der zweiten Bewegungseinrichtung sein. Die zweite Bewegung der zweiten Bewegungseinrichtung kann dazu führen, dass der Schlauchverschluss in den Schlauch gepresst wird. Beispielsweise kann ein Presselement zum Ende des Schlauchs bewegt werden, um einen Stopfen einzupressen. Insbesondere erfolgt zuvor erste Bewegung der ersten Bewegungseinrichtung. Ein Presselement der Verschlusseinrichtung ist insbesondere parallel zur Längsachse des Kanals und/oder des Schlauchs ausgerichtet.

Freigeben des Schlauchs meint Lösen des Schlauchs aus dem mittels der Halteeinrichtung gehaltenen Zustand. Der freigegebene Schlauch kann anschließend aus der Vorrichtung hinausbewegt werden bzw. die Vorrichtung kann von dem freigegebenen Schlauch entfernt werden.

Ein Teil der Halteeinrichtung kann mit der zweiten Bewegungseinrichtung in Wirkverbindung stehen. Die zweite Bewegung der zweiten Bewegungseinrichtung kann dazu führen, dass der Schlauch freigegeben wird. Beispielsweise kann ein Keil vorhanden sein, um zwei Schenkel der Halteeinrichtung auseinander zu bewegen, um den Schlauch freizugeben. Die zweite Bewegungseinrichtung kann in Wirkverbindung mit dem Keil stehen oder den Keil umfassen.

Werden sowohl das Verschließen als auch das Freigeben durch die zweite Bewegung ausgelöst, sind der Teil der Verschlusseinrichtung und der Keil bevorzugt so angeordnet, dass während der zweiten Bewegung zuerst der Teil der Verschlusseinrichtung das Ende verschließt und erst danach der Keil mit der Halteeinrichtung in Kontakt kommt, um den Schlauch freizugeben. Die Zielposition wird erst erreicht, wenn der Schlauch freigegeben ist. Es werden zwei Schritte durch eine einzige Betätigung des Benutzers durchgeführt. So können das Verschließen und das Freigeben besonders einfach und schnell erfolgen.

Sind die erste Bewegungseinrichtung und die zweite Bewegungseinrichtung vorhanden, kann mit zwei einfachen Schritten das Halten, Schneiden, Verschließen und Freigeben des Schlauchs erfolgen. Dies ermöglicht ein besonders einfaches Verfahren.

In einer Ausgestaltung ist die Vorrichtung so eingerichtet, dass die erste Bewegungseinrichtung lediglich die erste Bewegung ausführen kann, wobei jede andere Bewegung der ersten Bewegungseinrichtung blockiert ist.

Beispielsweise ist vor oder nach der ersten Bewegung eine Bewegung in entgegengesetzter Richtung blockiert. So kann eine Fehlbedienung ausgeschlossen werden. Auch eine mehrmalige Ausführung der ersten Bewegung ist bevorzugt blockiert. Die erste Bewegung kann eine geführte Bewegung sein. Es können eine oder mehrere Führungselemente wie z. B. Nuten vorhanden sein, entlang denen korrespondierende Formelemente der ersten Bewegungseinrichtung bewegt werden können. Jede Bewegung, die nicht entlang der Führungselemente erfolgt, ist so blockiert. Es kann ein erster Anschlag vorhanden sein, der ein Bewegen der ersten Bewegungseinrichtung entgegen der Richtung der ersten Bewegung blockiert. Es kann ein zweiter Anschlag vorhanden sein, der ein Bewegen über das Ende der ersten Bewegung hinaus verhindert. Es kann ein erster Rastmechanismus vorhanden sein, der die erste Bewegungseinrichtung in ihrer Zielposition verrastet und somit dort hält.

In einer Ausgestaltung ist die Vorrichtung so eingerichtet, dass die zweite Bewegungseinrichtung lediglich die zweite Bewegung ausführen kann, wobei jede andere Bewegung der zweiten Bewegungseinrichtung blockiert ist.

Die zweite Bewegungseinrichtung kann lediglich die zweite Bewegung ausführen. Beispielsweise ist nach der zweiten Bewegung eine Bewegung in entgegengesetzter Richtung blockiert. So kann eine Fehlbedienung ausgeschlossen werden. Auch eine mehrmalige Ausführung der zweiten Bewegung ist bevorzugt blockiert. Die zweite Bewegung kann eine geführte Bewegung sein. Es können eine oder mehrere Führungselemente wie z. B. Nuten vorhanden sein, entlang denen korrespondierende Formelemente der zweiten Bewegungseinrichtung bewegt werden können. Jede Bewegung, die nicht entlang der Führungselemente erfolgt, ist so blockiert. Es kann ein dritter Anschlag vorhanden sein, der ein Bewegen der zweiten Bewegungseinrichtung entgegen der Richtung der zweiten Bewegung blockiert. Es kann ein vierter Anschlag vorhanden sein, der ein Bewegen über das Ende der zweiten Bewegung hinaus verhindert. Es kann ein zweiter Rastmechanismus vorhanden sein, der die zweite Bewegungseinrichtung in ihrer Zielposition verrastet und somit dort hält.

In einer Ausgestaltung ist die Vorrichtung so eingerichtet, dass die zweite Bewegung der zweiten Bewegungseinrichtung erst ausgeführt werden kann, nachdem die erste Bewegung der ersten Bewegungseinrichtung ausgeführt worden ist.

Insbesondere kann die zweite Bewegung erst begonnen werden, nachdem der Schlauch durchtrennt worden ist und/oder nachdem die erste Bewegungseinrichtung ihre Zielposition erreicht hat. Auf diese Weise kann eine Fehlbedienung besonders zuverlässig verhindert werden.

In einer Ausführungsform umfasst die Vorrichtung einen Blockiermechanismus, welcher die zweite Bewegung der zweiten Bewegungseinrichtung so lange blockiert, bis der Schlauch durchtrennt worden ist. Beispielsweise kann die erste Bewegungseinrichtung derart in Wirkverbindung mit dem Blockiermechanismus stehen, dass die Blockade freigegeben wird, wenn zumindest ein Teil der Klinge eine vorgegebene Position erreicht hat, die sich entlang der Bewegungsrichtung der Klinge beispielsweise hinter dem Schlauch befindet.

In einer Ausgestaltung weist die Vorrichtung einen Kanal auf, in welchen der Schlauch eingeführt werden kann, um mit der Halteeinrichtung gehalten zu werden, um mit der Verschlusseinrichtung verschlossen zu werden und/oder um mit der Trennvorrichtung durchtrennt zu werden.

Der Kanal ist ein länglicher Hohlraum zur Aufnahme des Schlauchs. Der Kanal ist entlang seiner Längsrichtung zumindest bereichsweise offen und/oder zumindest bereichsweise geschlossen. Der Kanal kann einen kreiszylinderförmigen Querschnitt aufweisen. Der Schlauch kann in den Kanal eingelegt oder eingeschoben werden. Bevorzugt ist der Kanal lediglich an einer oder beiden Stirnseiten offen, so dass der Schlauch in den Kanal eingeschoben wird. Es kann an der Außenseite der Vorrichtung eine Markierung und/oder ein Sichtfenster vorhanden sein, um die Position der Klinge sichtbar zu machen, insbesondere in Bezug zum Kanal. Es kann dann der Schlauch so weit in den Kanal eingeschoben oder durch den Kanal durchgeschoben werden, bis sich das herzustellende und zu verschließende Ende im Bereich der Markierung befindet. Anschließend kann der Schlauch durchtrennt und verschlossen werden.

In einer Ausgestaltung weist die Halteeinrichtung zwei Schenkel auf, zwischen denen der Schlauch kraftschlüssig und/oder formschlüssig gehalten werden kann. Die Halteeinrichtung kann in dieser Ausgestaltung als Gabel bezeichnet werden.

Die Schenkel werden insbesondere senkrecht zum Schlauch bewegt. Ein Zwischenraum zwischen den Schenkeln kann sich verjüngen, so dass ein Einführen des Schlauchs in den Zwischenraum erleichtert wird und/oder so dass bei Bewegung der Schenkel relativ zum Schlauch eine Position erreicht wird, in der der Schlauch gehalten wird. Die Schenkel können unterschiedliche Abschnitte eines einstückigen Bauteils sein. Eine solche Ausgestaltung der Halteeinrichtung kann den Schlauch klemmen und demnach auch als Klemmeinrichtung bezeichnet werden.

An der dem Schlauch zugewandten Seite eines oder beider Schenkel befindet sich typischerweise eine Kontaktfläche, die den Schlauch kontaktiert und so hält. Die Kontaktfläche kann eine glatte oder strukturierte Oberfläche aufweisen. Die Kontaktfläche kann für eine kraftschlüssiges Halten eingerichtet sein. Die Kontaktfläche kann zudem ein oder mehrere Formschlusselemente aufweisen, beispielsweise Rastelemente oder Haken, die in die Außenwand des Schlauchs greifen und so einen formschlüssigen Schutz gegen ein Herausbewegen des Stopfens aus dem Schlauch bieten können. Die Formschlusselemente sind typischerweise so angeordnet, dass sie ein Bewegen des Schlauchs aus der Vorrichtung heraus entlang seiner Längsachse verhindern. Die Formschlusselemente sind demnach typischerweise quer zur Längserstreckung der Schenkel angeordnet. Grundsätzlich kann die Halteeinrichtung die Formschlusselemente allerdings auch unabhängig von ihrer Ausgestaltung mit zwei Schenkeln aufweisen. Beispielsweise kann eine beliebig geformte Haltefläche der Halteeinrichtung die Formschlusselemente aufweisen.

Die Halteeinrichtung kann ein Widerlager aufweisen, um den Schlauch gegen die Bewegung der Schenkel zu stützen. Das Widerlager ist typischerweise mit dem Grundkörper verbunden. Die Schenkel werden so relativ zum Widerlager und zum Schlauch bewegt. Auf diese Weise wird verhindert, dass der Schlauch durch die Schenkel umgebogen wird. Das Halten kann dadurch verbessert werden. Das Widerlager kann so angeordnet sein, dass es sich in der Zielposition der Schenkel zumindest abschnittsweise zwischen den Schenkeln befindet. Das Widerlager kann einen Kontaktbereich zum Kontaktieren des Schlauchs aufweisen. Der Kontaktbereich kann der äußeren Form des Schlauchs angepasst sein.

In einer Ausführungsform umfasst die Vorrichtung eine Freigabeeinrichtung zum Freigeben des in der Halteeinrichtung gehaltenen Schlauchs. Die Freigabeeinrichtung dient dazu, den Schlauch aus der mittels der Halteeinrichtung gehaltenen Position freizugeben. Die Freigabeeinrichtung kann in Wirkverbindung mit der zweiten Bewegungseinrichtung stehen, um den Schlauch bei Ausführung der zweiten Bewegung freizugeben. Die Freigabeeinrichtung kann im Falle von zwei Schenkeln einen Keil umfassen.

In einer Ausgestaltung weist die Vorrichtung einen Keil auf, welcher zwischen die Schenkel bewegt werden kann. Auf diese Weise können die Schenkel auseinander bewegt werden, um den gehaltenen Schlauch freizugeben. Diese Ausgestaltung erlaubt ein besonders einfaches Freigeben der Schlauchs.

Ein Keil ist ein Bauteil mit einer oder zwei schrägen Oberflächen, um die Schenkel zu kontaktieren. Es wird mit fortschreitender Bewegung des Keils eine steigende, auswärts gerichtete Kraft auf einen oder beide Schenkel ausgeübt. Der Keil kann etwa symmetrisch ausgebildet sein, um beide Schenkel gleichermaßen zu bewegen. Typischerweise ist wenigstens ein Schenkel, bevorzugt beide Schenkel, derart flexibel, dass der Keil die Schenkel ausreichend auseinander bewegen kann, um den Schlauch freizugeben. Der Keil kann an einem Ende eines länglichen Elements angeordnet sein. Das längliche Element kann einen beliebigen Querschnitt aufweisen. Der Keil und/oder das längliche Element kann parallel zur Längsachse des Schlauchs ausgerichtet sein und/oder bewegt werden. Der Keil und/oder das längliche Element kann ungefähr oder genau senkrecht zur Erstreckungsrichtung der Schenkel ausgerichtet sein. Der Keil und insbesondere auch das längliche Element kann mit der zweiten Bewegungseinrichtung in Wirkverbindung stehen, um gemeinsam mit dieser bewegt zu werden.

In einer Ausgestaltung umfasst die Vorrichtung eine Baueinheit, die den Keil und das Presselement umfasst. Die Baueinheit ist insbesondere entlang der Längsachse des Schlauchs und/oder der Vorrichtung verschieblich. Keil und Presselement können fest miteinander verbunden sein, insbesondere einstückig hergestellt.

Die Baueinheit kann derart in Wirkverbindung mit der ersten Bewegungseinrichtung stehen, dass sie die erste Bewegung der ersten Bewegungseinheit mit ausführt. Beispielsweise kann ein den Keil aufweisendes längliches Element so im zweiten Kanal angeordnet sein, dass die erste Bewegungseinrichtung die Baueinheit mitführt. Alternativ oder ergänzend kann das Presselement so im ersten Kanal angeordnet sein, dass die erste Bewegungseinrichtung die Baueinheit mitführt. Die Baueinheit kann also mit der ersten Bewegungseinrichtung quer zur Erstreckungsrichtung des Presselements und/oder des Schlauchs bewegt werden. So kann das Presseelement an dem abgeschnittenen Ende des Schlauchs ausgerichtet werden.

Die Baueinheit kann derart in Wirkverbindung mit der zweiten Bewegungseinrichtung stehen, dass sie die zweite Bewegung der zweiten Bewegungseinrichtung mit dieser ausführt. Beispielsweise umfasst die Baueinheit an einer dem Keil und dem Presselement abgewandten Seite eine Kontaktfläche und die zweite Bewegungseinrichtung umfasst an der der Baueinheit zugewandten Seite eine Schubfläche. Die Schubfläche kann dann bei der Durchführung der zweiten Bewegung die Kontaktfläche derart nach vorn schieben, dass die Baueinheit sich mit bewegt. So kann das Presselement den Schlauchverschluss in den Schlauch pressen.

In einer Ausgestaltung weist die Trennvorrichtung eine Klinge zum Durchtrennen des Schlauchs auf. In einer Ausgestaltung weist die Vorrichtung ein insbesondere mit der Klinge in Wirkverbindung stehendes Schubelement zum insbesondere seitlichen Schieben eines abgetrennten Schlauchstücks auf.

Das Schubelement ist insbesondere mit der Klinge verbunden, bevorzugt über ein weiteres Teil. Insbesondere sind die Klinge und das Schubelement an der ersten Bewegungseinrichtung angeordnet oder können mit dieser gekoppelt werden. Das Schubelement schiebt das abgetrennte Schlauchstück aus seiner ursprünglichen Position weg. Insbesondere wird das Schlauchstück quer zur Längserstreckung des Schlauchs verschoben. So wird das zu verschließende Ende des Schlauchs freigegeben. Auf diese Weise kann das Verschließen besonders einfach erfolgen. Die Klinge ist bevorzugt aus Metall hergestellt.

Insbesondere ist ein Gegenelement vorhanden, welches mit dem Grundkörper verbunden ist. Die Klinge wird dann relativ zum Schlauch und zum Gegenelement bewegt. Das Gegenelement kann ein seitliches Verschieben des Schlauchs durch die Klinge verhindern. Das Gegenelement kann in Bezug zur Bewegungsrichtung der Klinge schräg angeordnet sein, um einen besonders einfachen, definierten Schnitt zu ermöglichen.

In einer Ausgestaltung ist die erste Bewegungseinrichtung zweiteilig ausgebildet. Ein erster Teil der ersten Bewegungseinrichtung kann die Halteeinrichtung umfassen. Alternativ oder ergänzend kann ein zweiter Teil der ersten Bewegungseinrichtung die Trennvorrichtung und/oder die Aufbewahrungseinrichtung umfassen.

Der erste Teil und der zweite Teil sind miteinander verbunden und können gemeinsam die erste Bewegung ausführen. Der erste Teil und der zweite Teil können einstückig hergestellt sein. Zwischen dem ersten Teil und dem zweiten Teil kann sich in der Ausgangsposition und/oder in der Zielposition ein Bereich des Grundkörpers befinden.

In einer Ausführungsform enthält die erste Bewegungseinrichtung einen ersten Kanal. Der erste Kanal kann im zweiten Teil der ersten Bewegungseinrichtung angeordnet sein. Der erste Kanal ist, wenn die erste Bewegungseinrichtung in der Ausgangsposition ist, insbesondere parallel, aber axial versetzt zum Kanal der Vorrichtung ausgerichtet. Der erste Kanal ist, wenn die erste Bewegungseinrichtung in der Zielposition ist, insbesondere fluchtend mit dem Kanal der Vorrichtung ausgerichtet. Der erste Kanal der ersten Bewegungseinrichtung kann die Aufbewahrungseinrichtung zur Aufbewahrung des Schlauchverschlusses darstellen. Der erste Kanal kann einen kreiszylinderförmigen Querschnitt aufweisen.

In einer Ausführungsform enthält die erste Bewegungseinrichtung zweiten Kanal. Der zweite Kanal kann im zweiten Teil der ersten Bewegungseinrichtung angeordnet sein. Der zweite Kanal ist insbesondere sowohl in der Ausgangsposition als auch in der Zielposition der ersten Bewegungseinrichtung parallel, aber axial versetzt zum Kanal der Vorrichtung ausgerichtet. Ist ein erster Kanal vorhanden, sind der erste Kanal und der zweite Kanal typischerweise parallel, aber voneinander beabstandet angeordnet. Der zweite Kanal dient insbesondere zur Aufnahme und/oder Durchführung eines Keils, der Schenkel der Halteeinrichtung auseinander bewegt, wie weiter unten beschrieben, und/oder eines den Keil aufweisenden länglichen Elements. Der zweite Kanal kann einen kreiszylinderförmigen Querschnitt aufweisen. Der Querschnitt des zweiten Kanals kann größer sein als der Querschnitt des ersten Kanals.

In einer Ausführungsform umfasst die Vorrichtung, insbesondere der Grundkörper, ein Sperrelement, welches den ersten Kanal axial verschließt, bevorzugt in Richtung zur Klinge. Das Sperrelement kann sich an dem zwischen den zwei Teilen der ersten Bewegungseinrichtung befindlichen Bereich des Grundkörpers angeordnet sein. Das Blockieren erfolgt insbesondere, wenn sich die erste Bewegungseinrichtung in der Ausgangsposition befindet. An der gegenüberliegenden axialen Seite kann der erste Kanal ebenfalls verschlossen sein, beispielsweise mittels des Schubelements. Durch das Blockieren kann der Schlauchverschluss nicht herausfallen. Die Blockierung kann bevorzugt nur durch Bewegen der ersten Bewegungseinrichtung aufgehoben werden.

In einer Ausführungsform definiert die zweite Bewegungseinrichtung und/oder der Grundkörper der Vorrichtung einen dritten Kanal. Der dritte Kanal ist insbesondere axial fluchtend zum Kanal der Vorrichtung ausgerichtet bzw. stellt eine Verlängerung des Kanals der Vorrichtung dar. Der Schlauch kann insbesondere entlang einer Geraden durch den Kanal der Vorrichtung und den dritten Kanal geführt werden. Insbesondere weist der dritte Kanal, zumindest in einem dem Kanal der Vorrichtung zugewandten Bereich, Querschnitt eines Langlochs bzw. Ovals auf. Alternativ oder ergänzend kann der Kanal der Vorrichtung zwischen der zweiten Bewegungseinrichtung und der ersten Bewegungseinrichtung einen derartigen Querschnitt aufweisen. Auf diese Weise kann der abgeschnittene Schlauch mit dem Schubelement innerhalb des jeweiligen Kanals quer zu seiner Längserstreckung verschoben werden und in einer Position gelagert werden, die nicht mit dem zu verschließenden Schlauch fluchtet.

In einer Ausführungsform ist der Grundkörper, die erste Bewegungseinrichtung und/oder die zweite Bewegungseinrichtung aus Kunststoff hergestellt oder umfasst zumindest Kunststoff. In einer Ausführungsform ist ein Gehäuse der Vorrichtung aus Kunststoff hergestellt. In einer Ausführungsform ist die gesamte Vorrichtung mit Ausnahme weniger Elemente wie z. B. der Klinge aus Kunststoff hergestellt. Insbesondere wird ein thermoplastischer Kunststoff verwendet.

Ein weiterer Aspekt der Erfindung ist ein System, umfassend eine erfindungsgemäße Vorrichtung und einen medizinischen Schlauch. Insbesondere umfasst das System zudem den Schlauchverschluss.

Ein weiterer Aspekt der Erfindung ist eine Verwendung einer Vorrichtung zum Verschließen eines medizinischen Schlauchs. Insbesondere ist die Vorrichtung eine erfindungsgemäße Vorrichtung. Bevorzugt wird die Vorrichtung vor dem Verschließen zum Schneiden des Schlauchs verwendet. Alle Merkmale, Vorteile und Ausgestaltungen der eingangs beschriebenen Vorrichtung gelten ebenso für die Verwendung und umgekehrt.

Ein weiterer Aspekt der Erfindung ist ein Verfahren zum Verschließen eines medizinischen Schlauchs mit einer insbesondere erfindungsgemäßen Vorrichtung. Das Verfahren kann das Durchtrennen des in der Halteeinrichtung der Vorrichtung gehaltenen Schlauchs mit einer Trennvorrichtung der Vorrichtung umfassen. Das Verfahren kann ferner das Verschließen des in der Halteeinrichtung der Vorrichtung gehaltenen Schlauchs mit einem in der Aufbewahrungseinrichtung der Vorrichtung befindlichen Schlauchverschluss umfassen. Alle Merkmale, Vorteile und Ausgestaltungen der eingangs beschriebenen Vorrichtung sowie der Verwendung gelten ebenso für das Verfahren und umgekehrt.

Das Verfahren kann einige oder alle der folgenden Schritte in beliebiger Kombination und Reihenfolge umfassen:
- Einführen des Schlauchs in den Kanal,
- Halten des Schlauchs durch die Halteeinrichtung,
- Durchtrennen des Schlauchs mittels der Trennvorrichtung,
- Bewegen des abgetrennten Schlauchstücks mittels eines Schubelements,
- Verschließen des Schlauchs mit der Verschlusseinrichtung, insbesondere durch Einpressen eines Stopfens, insbesondere aus der Aufbewahrungseinrichtung,
- Insbesondere Lösen des gehaltenen Schlauchs,
- Herausziehen des Schlauchs mitsamt der Verschlusseinrichtung.

Insbesondere erfolgt das Einpressen derart, dass der Stopfen nicht über das hergestellte Ende des Schlauchs hinausragt, beispielsweise bündig mit dem Ende des Schlauchs ist.

Die Bedienung der Vorrichtung kann einige oder alle der folgenden Schritte in beliebiger Kombination umfassen:
- Einführen des Schlauchs in den Kanal,
- Durchführen der ersten Bewegung der ersten Bewegungseinrichtung,
- Durchführen der zweiten Bewegung der zweiten Bewegungseinrichtung,
- Herausziehen des Schlauchs mitsamt der Verschlusseinrichtung.

Nachfolgend werden Ausführungsbeispiele der Erfindung auch anhand von Figuren näher erläutert. Merkmale der Ausführungsbeispiele können einzeln oder in einer Mehrzahl mit den beanspruchten Gegenständen kombiniert werden, sofern nichts Gegenteiliges angegeben ist. Die beanspruchten Schutzbereiche sind nicht auf die Ausführungsbeispiele beschränkt.

Es zeigen:
- Figur 1:: eine perspektivische Zeichnung einer Vorrichtung,
- Figuren 2 und 3:: geschnittene perspektivische Zeichnungen einer Vorrichtung,
- Figur 4:: eine Schnittzeichnung einer Vorrichtung,
- Figuren 5 und 6:: geschnittene perspektivische Zeichnungen einer Vorrichtung,
- Figur 7:: eine Schnittzeichnung einer Vorrichtung,
- Figuren 8 und 9:: teilgeschnittene perspektivische Zeichnungen einer Vorrichtung bei der Verwendung,
- Figur 10:: eine Schnittzeichnung einer Vorrichtung bei der Verwendung,
- Figur 11:: geschnittene perspektivische Zeichnungen einer Vorrichtung bei der Verwendung, sowie
- Figur 12:: eine weitere Schnittzeichnung einer Vorrichtung bei der Verwendung

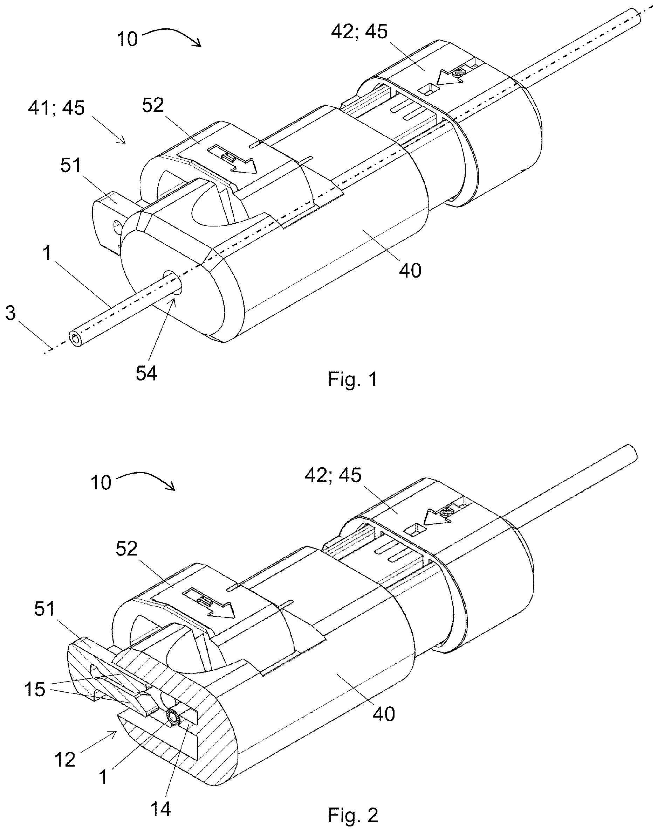

Figur 1 zeigt eine erfindungsgemäße Vorrichtung 10. Die Vorrichtung umfasst einen Grundkörper 40, eine erste Bewegungseinrichtung 41 und eine zweite Bewegungseinrichtung 41. Der Grundkörper umfasst einen Kanal 54, in dem ein medizinischer Schlauch 1 eingeführt worden ist. Der Kanal 54 erstreckt sich entlang einer Längsachse 3. Ist von einer axialen Richtung etc. die Rede, wird Bezug auf diese Längsachse genommen. Die erste Bewegungseinrichtung 41 kann senkrecht zur Längsachse 3 bewegt werden. Die Richtung der ersten Bewegung ist an der ersten Bewegungseinrichtung 41 mit einem Pfeil gekennzeichnet, der mit "1" beschriftet ist. Die erste Bewegungseinrichtung 41 umfasst zwei zusammenhängende Teile, und zwar einen ersten Teil 51 und einen zweiten Teil 52, die fest miteinander verbunden sind. Die zweite Bewegungseinrichtung 42 kann parallel zur Längsachse 3 bewegt werden. Die Richtung der zweiten Bewegung ist an der zweiten Bewegungseinrichtung 42 mit einem Pfeil gekennzeichnet, der mit "2" beschriftet ist.

Figur 2 zeigt eine ähnliche Darstellung, bei der der untere linke Bereich der Vorrichtung 10 abgeschnitten ist. Es zeigt sich, dass der erste Teil 51 der ersten Bewegungseinrichtung 41 zwei Schenkel 15 einer Halteeinrichtung 12 aufweist. Wenn die erste Bewegungseinrichtung 41 die erste Bewegung ausführt, bewegen sich die Schenkel 15 auf den Schlauch 1 zu und nehmen den Schlauch 1 zwischen sich auf. Ein Widerlager 14 ist am Grundkörper 40 der Vorrichtung befestigt oder ausgebildet und verhindert, dass der Schlauch 1 von den Schenkeln 15 seitlich weggedrückt wird. So kann der Schlauch 1 zwischen den Schenkeln 15 eingeklemmt und auf diese Weise gehalten werden. Der Schlauch 1 ist nun axial immobilisiert.

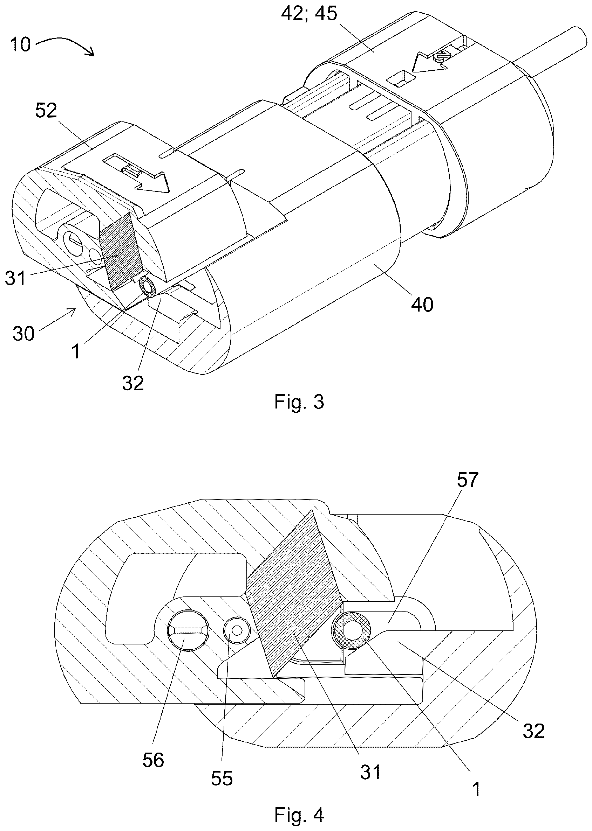

Figur 3 zeigt eine ähnliche Darstellung, bei der ein größerer Bereich der Vorrichtung 10 unten links abgeschnitten ist. Es zeigt sich, dass der zweite Teil 52 der ersten Bewegungseinrichtung 41 eine Klinge 31 einer Trennvorrichtung 30 aufweist. Wenn die erste Bewegungseinrichtung 41 die erste Bewegung ausführt, bewegt sich die Klinge 31 auf den Schlauch 1 zu und durchtrennt den Schlauch 1 senkrecht zur seiner Längsachse. Ein Gegenelement 32 ist am Grundkörper 40 der Vorrichtung befestigt oder ausgebildet und verhindert, dass der Schlauch 1 von der Klinge 31 seitlich weggedrückt wird. So kann der Schlauch 1 sauber geschnitten werden. Figur 4 zeigt die Form der Klinge 31 und des Gegenelements 32 in einer Schnittzeichnung mit axialer Blickrichtung.

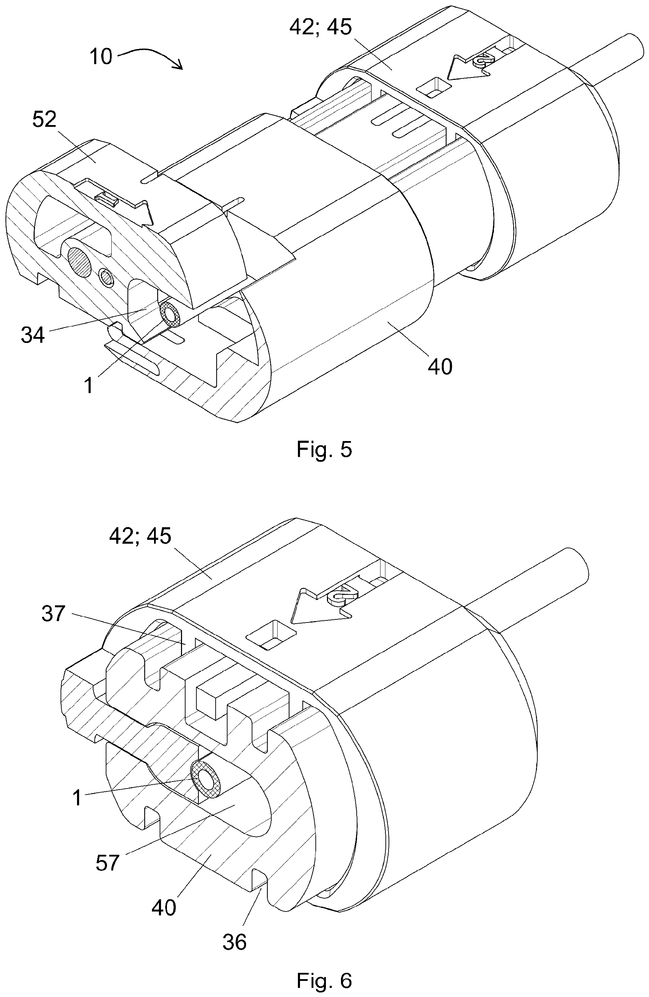

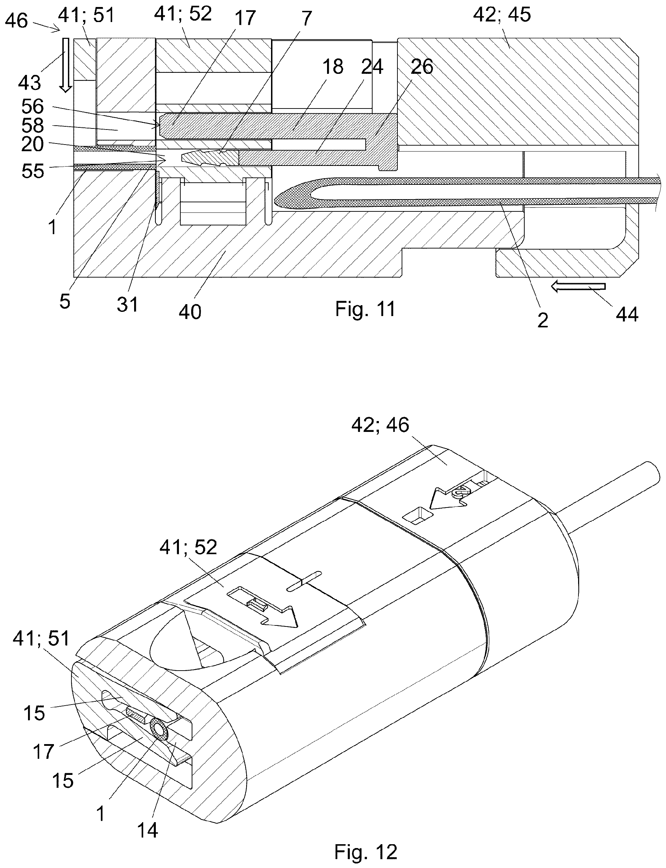

Figur 5 zeigt eine ähnliche Darstellung wie Figur 3, bei der ein noch größerer Bereich der Vorrichtung 10 unten links abgeschnitten ist. Es zeigt sich, dass der zweite Teil 52 der ersten Bewegungseinrichtung 41 axial hinter der Klinge 31 ein Schubelement 34 aufweist, welches als Formelement der ersten Bewegungseinrichtung 41 ausgeführt sein kann. Das Schubelement 34 dient dazu, das abgeschnittene Schlauchstück entlang der Richtung der ersten Bewegung zu schieben, um das abgeschnittene Ende des Schlauchs 1 freizugeben. Darauf wird in den Figuren 11 bis 13 näher eingegangen.

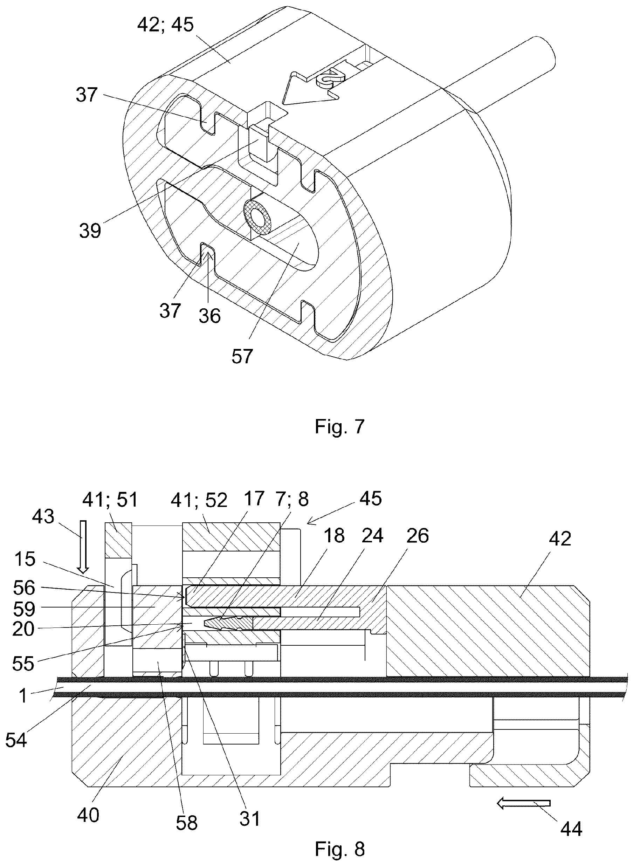

Die Figuren 6 und 7 zeigen ähnliche Darstellungen, bei der ein immer noch größerer Bereich der Vorrichtung 10 unten links abgeschnitten ist. Es zeigt sich, dass die zweite Bewegungseinrichtung 42 mehrere axial ausgerichtete längliche Formelemente 37 in Form von Stegen umfasst, die in korrespondierenden Nuten 36 des Grundkörpers verschieblich sind. Auf diese Weise kann eine geführte Bewegung der zweiten Bewegungseinrichtung 42 in Bezug zum Grundkörper 40 erfolgen. Die Bewegung der ersten Bewegungseinrichtung kann grundsätzlich auf dieselbe Weise erfolgen.

Im Bereich der zweiten Bewegungseinrichtung 42 befindet sich ein axial ausgerichteter dritter Kanal 57, der den Querschnitt eines Langlochs aufweist. Das abgetrennte Schlauchstück kann im dritten Kanal 57 wie beschrieben seitlich verschoben werden. In Figur 7 ist zudem ein zweiter Rastmechanismus 39 dargestellt, der die zweite Bewegungseinrichtung 42 nach Abschluss der zweiten Bewegung einrastet, sodass keine weitere Bewegung mehr möglich ist. Bevorzugt ist ein analog dazu aufgebauter erster Rastmechanismus (nicht dargestellt) vorhanden, der die erste Bewegungseinrichtung nach Abschluss der ersten Bewegung einrastet, sodass keine weitere Bewegung mehr möglich ist. Der erste Rastmechanismus und/oder der zweite Rastmechanismus 39 kann ein oder mehrere Rastelement aufweisen. Im Fall mehrerer Rastelemente kann an unterschiedlichen Positionen gerastet werden und so ein besonders sicheres Rasten bzw. eine besonders sichere Fixierung ermöglicht werden. Der erste Rastmechanismus und/oder der zweite Rastmechanismus 39 kann lösbar sein.

Figur 8 zeigt die Vorrichtung in einer von oben dargestellten Schnittzeichnung entlang der Ebene des Kanals 54, in dem sich der Schlauch 1 befindet. Gezeigt ist die Situation nach dem Einführen des Schlauchs 1, aber vor dem Ausführen der mit jeweiligen Pfeilen dargestellten ersten Bewegung 43 und zweiten Bewegung 44. Mit anderen Worten sind sowohl die erste Bewegungseinrichtung 41 als auch die zweite Bewegungseinrichtung 42 in ihrer jeweiligen Ausgangsposition. Um Wiederholungen zu vermeiden, wird auf die vorherigen Figuren und die jeweiligen Beschreibungen verwiesen.

Es ist erkennbar, dass der zweite Teil 52 der ersten Bewegungseinrichtung 41 einen ersten Kanal 55 aufweist, der als Aufbewahrungseinrichtung 20 für den Schlauchverschluss 7, dient, der hier beispielhaft als Stopfen 8 ausgeführt ist. Der erste Kanal 55 ist auf der rechten Seite durch das axial ausgerichtete Presselement 24 verschlossen. Der erste Kanal 55 ist auf der linken Seite durch das Sperrelement 59 verschlossen, welches der Bereich des Grundkörpers 40 ist, der sich in axialer Richtung zwischen dem ersten Teil 51 und dem zweiten Teil 52 befindet. So ist der Stopfen 8 verliersicher gehalten. In diesem Bereich des Grundkörpers 40 befindet sich zudem ein weiterer, axial ausgerichteter Kanal 58.

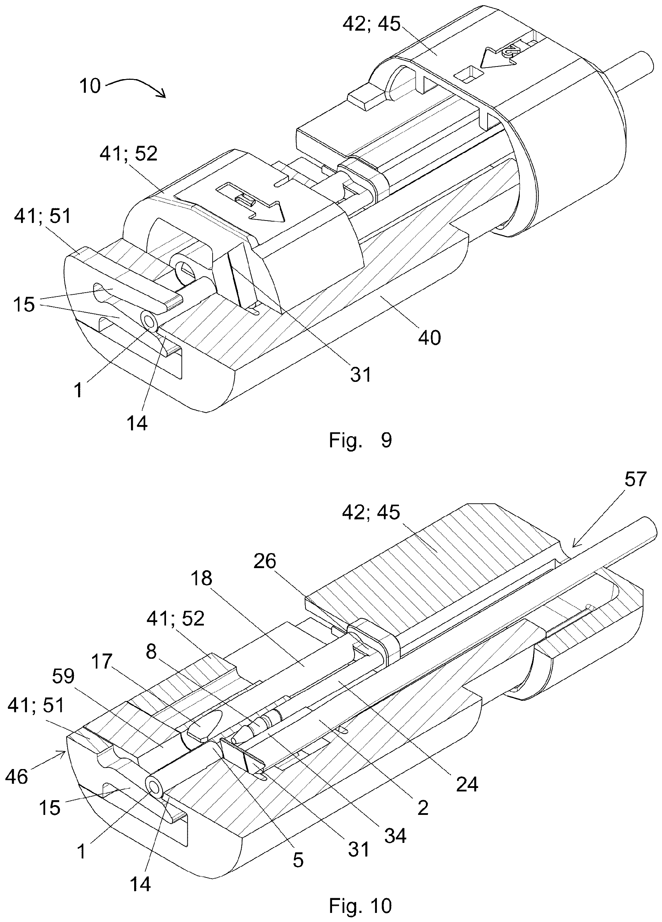

Der zweite Teil 52 umfasst ferner einen zweiten Kanal 56, in dem sich ein axial ausgerichtetes längliches Element 17 mit einem an seiner Spitze befindlichen Keil 17 befindet (siehe auch Figur 10). Längliches Element 18 und Keil 17 haben gemeinsam hier beispielhaft die Form wie Schaft und Spitze eines Schlitzschraubendrehers. Das längliche Element 18, der Keil 17 und das Presselement 24 sind als gemeinsame Baueinheit 26 ausgeführt, beispielsweise einstückig hergestellt.

Es ist erkennbar, dass bei Durchführung der ersten Bewegung 43 der Schlauch 1 mittels der Klinge 31 durchtrennt wird. Die Klinge 31 ist typischerweise so ausgebildet, dass sie bei Durchführen der ersten Bewegung vollständig durch den Schlauch bzw. die Position des Schlauchs vor dem Durchtrennen bewegt wird. Auf diese Weise kann das hergestellte Ende des Schlauchs freigegeben werden. Nach dem Durchtrennen erreichen die Schenkel 15 der Halteeinrichtung den Schlauch und greifen bzw. halten ihn. Der Ablauf der Verfahrensschritte wird in den folgenden Figuren dargestellt und im Folgenden erläutert.

In Figur 8 ist noch ein optionaler, wie folgt ausgebildeter Blockiermechanismus erkennbar: Die zweite Bewegung 44 der zweiten Bewegungseinrichtung 42 kann erst ausgeführt werden, nachdem die erste Bewegung 43 der ersten Bewegungseinrichtung 41 ausgeführt worden ist. Dies ist dadurch gewährleistet, dass in der dargestellten Ausgangsposition 45 der ersten Bewegungseinrichtung 41 der Keil 17 an das Sperrelement 59 stoßen würde, wenn die zweite Bewegungseinrichtung 42 entlang dem Pfeil 44 bewegt würde. Die zweite Bewegungseinrichtung würde die Baueinheit 26, umfassend das längliche Element 18 mit dem Keil 17 sowie das Presselement 24, nach links bewegen und damit den Keil 17 an das Sperrelement 59 drücken. Erst wenn die Baueinheit 26 gemeinsam mit der ersten Bewegungseinrichtung 41 entlang der Richtung des Pfeils 43 bewegt worden ist, fluchten der zweite Kanal 56 und der Kanal 58 im Grundkörper 40, sowie der erste Kanal 55 und der Kanal 54 im Grundkörper (siehe Figuren 8 und 11).

Die Figuren 9 bis 11 zeigen den Zustand nach Ausführung der ersten Bewegung. Die erste Bewegungseinrichtung 41 befindet sich in ihrer Zielposition 46. Die Klinge 31 hat den Schlauch 1 passiert und gibt so das hergestellte Ende 5 des Schlauchs frei. Der gekürzte Schlauch wird von der Halteeinrichtung, genauer von den Schenkeln 15 und dem Gegenelement 14, gehalten. Es ist erkennbar, dass das abgetrennte Schlauchstück 2 durch das Schubelement 34 im dritten Kanal 57 parallel zur Längsachse verschoben worden ist und so aus der Achse des gekürzten Schlauchs 1 wegbewegt worden ist. Der zweite Teil 52 hat die Baueinheit 26 mitbewegt, so dass der Kanal 59 mit dem länglichen Element 18 und dem Keil 17 fluchtet. Zudem fluchten das Presselement 24 und der Stopfen 8 mit dem gekürzten Schlauch 1.

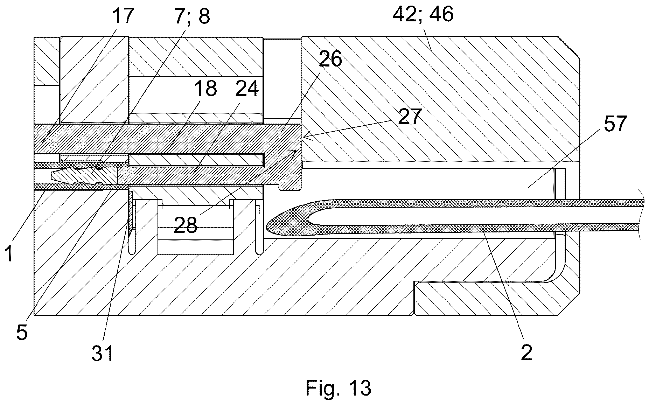

Die Figuren 12 und 13 zeigen den Zustand nach Ausführung der zweite Bewegung 44. Auch die zweite Bewegungseinrichtung 42 befindet sich nun in ihrer Zielposition 46. Insbesondere in Figur 13 ist erkennbar, dass eine Schubfläche 28 der zweiten Bewegungseinrichtung 42 eine Kontaktfläche 27 der Baueinheit 26 kontaktiert und so die Baueinheit 26 mit in axialer Richtung verschoben hat. Dabei ist zunächst mittels des Presselements 24 der Stopfen 8 in das hergestellte Ende 5 des Schlauchs gepresst worden. Anschließend, bei Fortführung der zweiten Bewegung, ist der Keil 17 in Kontakt mit mindestens einem Schenkel 15 gelangt, um die Schenkel 15 auseinander zu bewegen. Dies ist in Figur 12 erkennbar. Auf diese Weise ist der Schlauch 1 wieder freigegeben worden und kann nun, mitsamt des eingepressten Stopfens 8, in axialer Richtung aus der Vorrichtung gezogen werden.

Sowohl die erste Bewegungseinrichtung 41 als auch die zweite Bewegungseinrichtung 42 ist nun verrastet, so dass keine weitere Bewegung mehr möglich ist. So kann eine Fehlbedienung verhindert werden. Insbesondere ist die Vorrichtung für den einmaligen Gebrauch vorgehsehen.

**Bezugszeichenliste**

| | |
|---|---|
| Schlauch | 1 |
| Schlauchstück | 2 |
| Längsachse | 3 |
| Ende | 5 |
| Schlauchverschluss | 7 |
| Stopfen | 8 |
| Vorrichtung | 10 |
| Halteeinrichtung | 12 |
| Widerlager | 14 |
| Schenkel | 15 |
| Keil | 17 |
| Längliches Element | 18 |
| Aufbewahrungseinrichtung | 20 |
| Verschlusseinrichtung | 22 |
| Presselement | 24 |
| Baueinheit | 26 |
| Kontaktfläche | 27 |
| Schubfläche | 28 |
| Trennvorrichtung | 30 |
| Klinge | 31 |
| Gegenelement | 32 |
| Schubelement | 34 |
| Nut | 36 |
| Formelement | 37 |
| Zweiter Rastmechanismus | 39 |
| Grundkörper | 40 |
| erste Bewegungseinrichtung | 41 |
| zweite Bewegungseinrichtung | 42 |
| erste Bewegung | 43 |
| zweite Bewegung | 44 |
| Ausgangsposition | 45 |
| Zielposition | 46 |
| Erster Teil | 51 |
| Zweiter Teil | 52 |
| Kanal | 54 |
| Erster Kanal | 55 |
| Zweiter Kanal | 56 |
| Dritter Kanal | 57 |
| Kanal | 58 |
| Sperrelement | 59 |

## Patentansprüche

1. Vorrichtung (10) zum Verschließen eines medizinischen Schlauchs (1), umfassend eine Halteeinrichtung (12) zum Halten eines medizinischen Schlauchs (1), eine Aufbewahrungseinrichtung (20) zum Aufbewahren eines Schlauchverschlusses (7) sowie eine Verschlusseinrichtung (22) zum Verschließen des in der Halteeinrichtung (12) gehaltenen Schlauchs (1) mit einem in der Aufbewahrungseinrichtung (20) befindlichen Schlauchverschluss (7).

2. Vorrichtung (10) nach dem vorhergehenden Anspruch, wobei die Vorrichtung (10) ferner eine Trennvorrichtung (30) zum Durchtrennen des Schlauchs (1) aufweist, wobei die Verschlusseinrichtung (22) dazu eingerichtet ist, ein mit der Trennvorrichtung (30) hergestelltes Ende (5) des Schlauchs (1) zu verschließen.

3. Vorrichtung (10) nach einem der vorhergehenden Ansprüche, wobei die Verschlusseinrichtung (22) ein Presselement (24) zum Pressen des Schlauchverschlusses (7) in den Schlauch (1) aufweist.

4. Vorrichtung (10) nach einem der vorhergehenden Ansprüche, wobei die Vorrichtung (10) eine erste Bewegungseinrichtung (41) aufweist, wobei die Vorrichtung (10) so eingerichtet ist, dass mit einer ersten Bewegung (43) der ersten Bewegungseinrichtung (41) das Halten des Schlauchs (1) sowie das Durchtrennen des Schlauchs (1) ausgelöst wird.

5. Vorrichtung (10) nach einem der vorhergehenden Ansprüche, wobei die Vorrichtung (10) eine zweite Bewegungseinrichtung (42) aufweist, wobei die Vorrichtung (10) so eingerichtet ist, dass mit einer zweiten Bewegung (44) der zweiten Bewegungseinrichtung (42) das Verschließen des Schlauchs (1) sowie ein Freigeben des Schlauchs (1) ausgelöst wird.

6. Vorrichtung (10) nach einem der Ansprüche 4 und 5, wobei die Vorrichtung (10) so eingerichtet ist, dass die erste Bewegungseinrichtung (41) lediglich die erste Bewegung (43) ausführen kann, wobei jede andere Bewegung der ersten Bewegungseinrichtung (41) blockiert ist, und/oder wobei die Vorrichtung (10) so eingerichtet ist, dass die zweite Bewegungseinrichtung (42) lediglich die zweite Bewegung (44) ausführen kann, wobei jede andere Bewegung der zweiten Bewegungseinrichtung (42) blockiert ist.

7. Vorrichtung (10) nach den Ansprüchen 4 und 5, wobei die Vorrichtung (10) so eingerichtet ist, dass die zweite Bewegung (44) der zweiten Bewegungseinrichtung (42) erst ausgeführt werden kann, nachdem die erste Bewegung (43) der ersten Bewegungseinrichtung (41) ausgeführt worden ist.

8. Vorrichtung (10) nach einem der vorhergehenden Ansprüche, wobei die Vorrichtung (10) einen Kanal (54) aufweist, in welchen der Schlauch (1) eingeführt werden kann, um mit der Halteeinrichtung (12) gehalten zu werden, um mit der Verschlusseinrichtung (22) verschlossen zu werden und/oder um mit der Trennvorrichtung (30) durchtrennt zu werden.

9. Vorrichtung (10) nach einem der vorhergehenden Ansprüche, wobei die Halteeinrichtung (12) zwei Schenkel (15) aufweist, zwischen denen der Schlauch (1) kraftschlüssig und/oder formschlüssig gehalten werden kann.

10. Vorrichtung (10) nach dem vorhergehenden Anspruch, wobei die Vorrichtung (10) einen Keil (17) aufweist, welcher zwischen die Schenkel (15) bewegt werden kann, um die Schenkel (15) auseinander zu bewegen und so den gehaltenen Schlauch (1) freizugeben.

11. Vorrichtung (10) nach dem vorhergehenden Anspruch und Anspruch 3, wobei die Vorrichtung (10) eine Baueinheit (26) aufweist, welche den Keil (17) und das Presselement umfasst, wobei die Baueinheit (26) entlang einer Längsachse (3) des Schlauchs (1) beweglich ist.

12. Vorrichtung (10) nach einem der vorhergehenden Ansprüche, wobei die Trennvorrichtung (30) eine Klinge (31) zum Durchtrennen des Schlauchs (1) aufweist, wobei die Vorrichtung (10) ein mit der Klinge (31) in Wirkverbindung stehendes Schubelement (34) zum Schieben eines abgetrennten Schlauchstücks (2) aufweist.

13. Vorrichtung (10) nach einem der Ansprüche 4 bis 10, wobei die erste Bewegungseinrichtung (41) zweiteilig ausgebildet ist, wobei ein erster Teil (55) der ersten Bewegungseinrichtung (41) die Halteeinrichtung (12) umfasst und ein zweiter Teil (56) der ersten Bewegungseinrichtung (41) die Trennvorrichtung (30) und/oder die Aufbewahrungseinrichtung (20) umfasst.

14. Verwendung einer Vorrichtung (10) gemäß einem der Ansprüche 1 bis 13 zum Verschließen eines medizinischen Schlauchs (1), wobei die Vorrichtung (10) vor dem Verschließen insbesondere zum Schneiden des Schlauchs (1) verwendet wird.

15. Verfahren zum Verschließen eines medizinischen Schlauchs (1) mit einer Vorrichtung (10) gemäß einem der Ansprüche 1 bis 13, umfassend:
- Optional Durchtrennen des in der Halteeinrichtung (12) der Vorrichtung (10) gehaltenen Schlauchs (1) mit einer Trennvorrichtung (30) der Vorrichtung (10),
- Verschließen des in der Halteeinrichtung (12) der Vorrichtung (10) gehaltenen Schlauchs (1) mit einem in der Aufbewahrungseinrichtung (20) der Vorrichtung (10) befindlichen Schlauchverschluss (7).
